# EUROPÄISCHE PATENTANMELDUNG

(11) **EP 4 101 302 A1**
(43) Veröffentlichungstag der Anmeldung: **14.12.2022**
(21) Anmeldenummer: 21213352.4
(22) Anmeldetag: 09.12.2021
(51) Int. Cl.: A23C 9/12, A23C 21/02, C12P 19/14, C07H 1/08, C07H 3/06, C12N 9/38, C08B 37/00

(54) **GALACTOOLIGOSACCHARIDE MIT VERMINDERTEN ANTEILEN AN MONOSACCHARIDEN UND LACTOSE**

(30) Priorität: 08.06.2021 EP 21178340
(71) Anmelder: DMK Deutsches Milchkontor GmbH, 27404 Zeven (DE)
(72) Erfinder: DÖRING, Sven-Rainer, 27404 Zeven (DE); STEFFENS, Marco, 27404 Elsdorf (DE)
(74) Vertreter: Fabry, Bernd

(57) **Zusammenfassung**

Vorgeschlagen werden Galactooligosaccharide mit verminderten Anteilen an Monosacchariden und Lactose, dadurch erhältlich oder erhalten, dass man
(i) eine wässrige Milchzuckerlösung bereitstellt,
(ii) die Milchzuckerlösung entkeimt,
(iii) den in der entkeimten Milchzuckerlösung aus Schritt (ii) vorhandenen Milchzucker unter Zugabe mindestens einer beta-Galactosidase innerhalb deren optimalen Temperatur- und pH-Wert-Intervalls über einen Zeitraum von mindestens 30 Minuten einer Transgalactosilierung unter Erhalt einer Reaktionsmischung unterwirft;
(iv) die in der Reaktionsmischung aus Schritt (iii) enthaltene Enzymmasse inhibiert;
(v) die Reaktionsmischung aus Schritt (iv) mit einer Lactosehydrolase und/oder einer Hefe, die Lactose zu CO₂ und Ethanol verstoffwechseln kann, nachbehandelt;
(vi) das nachbehandelte Produkt aus Schritt (v) einer Filtration unterwirft und
(vii) das so erhaltene Filtrat aus Schritt (vi) konfektioniert.

## Beschreibung

### GEBIET DER ERFINDUNG

Die vorliegende Erfindung befindet sich auf dem Gebiet der Kohlenhydrate und betrifft Galactooligosaccharide, die sich durch einen verminderten Gehalt an unerwünschten Nebenprodukten auszeichnen sowie ein Verfahren zu ihrer Herstellung.

### TECHNOLOGISCHER HINTERGRUND

Galactooligosaccharide (GOS) 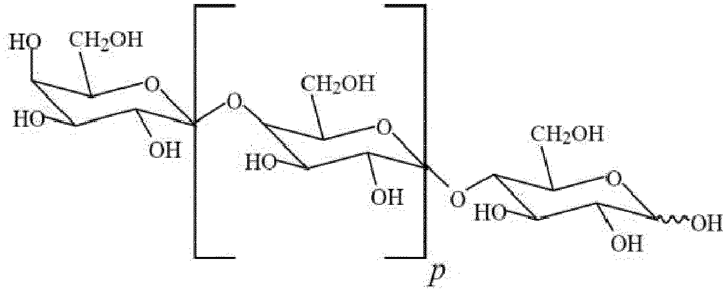 auch bekannt als Oligogalaktosyllactose, Oligogalaktose, Oligolaktose oder Transgalaktooligosaccharide (TOS), gehören zur Gruppe der Präbiotika. GOS kommt in handelsüblichen Produkten wie Nahrung für Säuglinge und Erwachsene vor.

Aufgrund der Konfiguration ihrer glykosidischen Bindungen widerstehen Galactooligosaccharide (GOS) weitgehend der Hydrolyse durch Speichel- und Darmverdauungsenzyme. Galactooligosaccharide werden daher als Präbiotika eingestuft, definiert als unverdauliche Nahrungsbestandteile, die sich vorteilhaft auf den Wirt auswirken, indem sie das Wachstum und/oder die Aktivität nützlicher Bakterien im Dickdarm stimulieren. Die erhöhte Aktivität dieser gesundheitsfördernden Bakterien führt zu einer Reihe von Effekten, sowohl direkt durch die Bakterien selbst als auch indirekt durch die organischen Säuren, die sie durch Fermentation produzieren. Beispiele für Wirkungen sind die Stimulierung der Immunfunktionen, die Aufnahme essentieller Nährstoffe, und die Synthese bestimmter Vitamine.

Galactooligosaccharide sind ein Substrat für Bakterien, wie Bifidobakterien und Laktobazillen. Studien mit Säuglingen und Erwachsenen haben gezeigt, dass mit Galakto-Oligosacchariden angereicherte Lebensmittel oder Getränke zu einer signifikanten Zunahme von Bifidobakterien führen. Diese Zucker kommen natürlich in der menschlichen Milch vor und sind als Oligosaccharide der menschlichen Milch bekannt. Beispiele sind Lakto-N-Tetraose, Lakto-N-Notetraose und Lakto-N-Fucopentaose.

Die menschliche Darmmikrobiota spielt eine Schlüsselrolle im intestinalen Immunsystem. Galacto-Oligosaccharide unterstützen die natürlichen Abwehrkräfte des menschlichen Körpers über die Darmmikroflora, indirekt, indem sie die Anzahl der Bakterien im Darm erhöhen und die Bindung oder das Überleben von *Escherichia coli, Salmonella Typhimurium* und Clostridien hemmen. GOS können das Immunsystem indirekt durch die Produktion von antimikrobiellen Substanzen positiv beeinflussen, indem sie die Vermehrung von pathogenen Bakterien reduzieren. Verstopfung ist ein potenzielles Problem, insbesondere bei Säuglingen, älteren Menschen und schwangeren Frauen. Bei Säuglingen kann die Fütterung mit Säuglingsnahrung mit Verstopfung und hartem Stuhl assoziiert sein. GOS können die Stuhlfrequenz verbessern und die mit der Verstopfung verbundenen Symptome lindern.

Die typische Gewinnung von GOS umfasst die folgenden Schritte:
1. Konzentrierung einer Milchzuckerlösung (Lactose, Sauermolke, Milchpermeat mit dem Ziel eine Trockenmasse von mindestens 30 Gew.-% zu erreichen;
2. Hocherhitzung/UHT-Behandlung zur Entkeimung bei 85-140 C über 90 bis 300 s;
3. Zugabe des Enzyms (z.B. *Aspergillus Oryzae*) und Einstellung der für das Enzym optimalen pH- und Temperaturbedingungen (z.B. pH =4,5, 55 C);
4. Verweilzeit in der Regel über 30 Minuten bis maximal 24 Stunden (abhängig vom Enzym und Enzymkonzentration), da ansonsten eine Rückspaltung erfolgt;
5. Thermische Inaktivierung des Enzyms beispielsweise durch Hochtemperaturpasteurisierung (90 °C, 10 min)
6. Aufreinigung, gegebenenfalls Konzentrierung, Sprühtrocknung.

Eine Übersicht zur Herstellung von Galactooligosacchariden findet sich auch von *K. Zerge* in einer Online-Publikation der Universität Dresden:
https://nbn-resolving.org/urn:nbn:de:bsz:14-qucosa-157121

### RELEVANTER STAND DER TECHNIK

EP 2620506 B1 (DUPONT) betrifft die Gewinnung von GOS ausgehend von Lactitol.

EP 3598901 B1 (HOCHSCHULE ANHALT) betrifft ein Verfahren zur Herstellung von GOS, bei dem man eine von L.bulgaricus (L.delbrueckii spp.bulgaricus) abgeleitete beta-Galactosidase bei einer Temperatur von 37 C oder weniger mit einer Lactose-haltigen Zusammensetzung wie Milch, Puffer oder Molke, z.B. Süßmolke, Sauermolke, Molkenkonzentrat oder Molkenpermeat, inkubiert.

EP 3041945 B1 (FRIESLAND) stellt ein Verfahren zur Herstellung von GOS aus Lactose bereit, das (i) das Inkontaktbringen einer Lactosebeschickung mit immobilisierter beta-Galactosidase (EC 3.2.1.23) und (ii) das Ermöglichen der GOS-Synthese umfasst, wobei die Lactosebeschickung eine wässrige Aufschlämmung von kristalliner Lactose ist.

WO 2008 037839 A1 (VALIO) bezieht sich auf ein Verfahren zur Herstellung von GOShaltigen Produkten auf Milchbasis durch Behandlung mit einer beta-Galactosidase.

WO 2018 048305 A1 (UNIV GRONINGEN) beschreibt die Verwendung einer GOS-Zusammensetzung, die verzweigte und lineare GOS-Spezies mit einem Polymerisationsgrad (DP) von 3 umfasst, wobei die verzweigten DP3-GOS-Spezies im Überschuss gegenüber den linearen DP3-GOS-Spezies vorhanden sind, zur Induktion von Mucin-Glykan-Verwertungswegen in nützlichen Darmbakterien in einem Tier.

WO 2018 210820 A1 (NOVOZYMES) beansprucht ein Verfahren, bei dem Milchsubstrat mit einem Laktosegehalt von mindestens 20 Gew.-% Laktose wird mit einem Enzym mit transgalaktosylierender Aktivität behandelt wird. Die transgalaktosylierende Aktivität des Enzyms kann durch Glykation von Lysin- und/oder Argininresten durch Inkubation des Enzyms mit hohen Glukosekonzentrationen bei erhöhten Temperaturen erhöht worden sein.

WO 2020 049016 A1 (FRIESLAND) bezieht sich auf das Gebiet der hypoallergenen Oligosaccharide zur Verwendung in Nahrungszusammensetzungen, insbesondere auf Oligosaccharide mit präbiotischen Eigenschaften. Es wird eine hypoallergene Oligosaccharid-Zusammensetzung bereitgestellt, die Galactooligosaccharide (GOS) umfasst, wobei (i) der Gehalt an Galactooligosacchariden (GOS) mindestens 40 Gew.-% der Gesamttrockensubstanz der Zusammensetzung beträgt; (ii) der Gehalt an Allolactose mindestens 10 Gew.-% der Gesamttrockensubstanz der Zusammensetzung beträgt; (iii) der Gehalt an 6'-GL mindestens 30 Gew.-% der Gesamt-GOS in der Zusammensetzung beträgt; und (iv) mindestens 0. 5 Gewichtsprozent des gesamten GOS einen Polymerisationsgrad (DP) von sechs oder mehr aufweisen.

WO 2020 117548 A1 (DUPONT) betrifft ein Verfahren zur Bereitstellung eines laktosearmen Produkts auf Milchbasis mit GOS-Fasern, bei dem ein Milchsubstrat mit Laktose mit einem transgalaktosylierenden Enzym behandelt wird, um GOS-Fasern und verbleibende Laktose bereitzustellen; Deaktivieren des transgalaktosylierenden Enzyms; Inkontaktbringen des Substrats auf Milchbasis mit GOS-Fasern mit einer Laktase, um die verbleibende Laktose abzubauen, um das laktosearme Produkt auf Milchbasis mit GOS-Fasern bereitzustellen, und Deaktivieren der Laktase.

WO 2020 141032 A1 (FRIESLAND) bezieht sich auf das Gebiet der Nahrungsmittelbestandteile, insbesondere auf wirtschaftlich attraktive Verfahren zur Herstellung von hypoallergenen Galactooligosacchariden (HA-GOS) und deren Verwendung in Nahrungs- und Futtermitteln. Es wird ein Verfahren zur Herstellung eines HA-GOS-Präparats bereitgestellt, das das Inkontaktbringen eines Lactose-Einsatzmaterials mit einer speziellen beta-Galactosidase (EC 3.2.1.23) umfasst, wobei das Lactose-Einsatzmaterial ein Käsemolkenpermeat (CWP) oder ein CWP ist, das mit Sialyllactose angereichert ist (SL-CWP).

### AUFGABE DER ERFINDUNG

GOS enthalten herstellungsbedingt Anteile an Rest-Lactose sowie geringe Mengen an Monosacchariden sowie GOS mit geringem DP (Degree of polymerisation), die aus anwendungstechnischen Gründen unerwünscht sind. Daher hat die Aufgabe der vorliegenden Erfindung darin bestanden, GOS zur Verfügung zu stellen, die gegenüber den Produkten des Stands der Technik eine verminderte Menge an diesen Stoffen aufweisen.

### BESCHREIBUNG DER ERFINDUNG

Ein erster Gegenstand der Erfindung betrifft Galactooligosaccharide mit verminderten Anteilen an Monosacchariden und Lactose, dadurch erhältlich oder erhalten, dass man
(i) eine wässrige Milchzuckerlösung bereitstellt,
(ii) die Milchzuckerlösung entkeimt,
(iii) den in der entkeimten Milchzuckerlösung aus Schritt (ii) vorhandenen Milchzucker unter Zugabe mindestens einer beta-Galactosidase innerhalb deren optimalen Temperatur-und pH-Wert-Intervalls über einen Zeitraum von mindestens 30 Minuten einer Transgalactosilierung unter Erhalt einer Reaktionsmischung unterwirft;
(iv) die in der Reaktionsmischung aus Schritt (iii) enthaltene Enzymmasse inhibiert;
(v) die Reaktionsmischung aus Schritt (iv) mit einer Lactosehydrolase und/oder einer Hefe, die Lactose zu CO₂ und Ethanol verstoffwechseln kann, nachbehandelt;
(vi) das nachbehandelte Produkt aus Schritt (v) einer Filtration unterwirft und
(vii) das so erhaltene Filtrat aus Schritt (vi) konfektioniert.

Ein weiterer Gegenstand der Erfindung betrifft ein entsprechendes Verfahren zur Herstellung von Galactooligosacchariden, umfassend die folgenden Schritte:
(i) Bereitstellen einer wässrigen Milchzuckerlösung;
(ii) Entkeimung der Milchzuckerlösung;
(iii) Transgalactosilierung der in der entkeimten Milchzuckerlösung aus Schritt (ii) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung;
(iv) Inhibierung der Enzymmasse in der Reaktionsmischung aus Schritt (iii)
(v) Nachbehandlung der Reaktionsmischung aus Schritt (iv) mit einer Lactosehydrolase und/oder einer Hefe, die Lactose zu CO₂ und Ethanol verstoffwechseln kann;
(vi) Filtration des nachbehandelten Produktes aus Schritt (v) sowie
(vii) Konfektionierung des Filtrates aus Schritt (iv).

In einer besonderen Ausführungsform zeichnet sich das erfindungsgemäße Verfahren dadurch aus, dass man
(a) die Enzymmasse durch Einstellen eines pH-Wertes außerhalb des Aktivitätsoptimums ganz oder teilweise inhibiert;
(b) die Reaktionsmischung zusammen mit der inhibierten Enzymmasse einer Filtration unter Erhalt eines Retentats (R1) und eines Permeats (P1) unterwirft;
(c) die inhibierte Enzymmenge als Retentat (R1) abtrennt und zum Schritt (iii) zurückführt; und
(d) das die Galactooligosaccharide enthaltende Permeat (P2) zum Schritt (v) weiterleitet.

Überraschenderweise wurde gefunden, dass sich durch Nachbehandlung der Transgalactosilierungsprodukte in der angegebenen Weise sowohl die Menge an noch im Produkt befindlicher Lactose als auch die unerwünschten Galactooligosaccharide mit niedrigem DP selektiv entfernen lassen, ohne dass es zum Abbau oder Abtrennung von den gewünschten GOS mit hohem DP kommt, da diese wegen ihrer Größe nicht von den Hefezellen aufgenommen werden können. Insbesondere liegt die Menge an Rest-Lactose sowie an Monosacchariden in den erfindungsgemäßen Produkten jeweils unterhalb der Zielgröße von 0,5 Gew.-% und insbesondere im Bereich von 0,1 bis 0,4 Gew.-%.

### Einsatzstoffe

Als Einsatzstoffe für die Herstellung von Galactooligosacchariden kommen Milchzuckerlösungen Frage, wie beispielsweise Lactoselösung, Sauermolke oder Milchpermeat. Wesentlich und gemeinsam ist den geeigneten Einsatzstoffen, dass sie eine ausreichende Menge an Lactose, speziell an glykosidisch gebundener Galactose besitzen.

Lactose, Milchzucker oder Laktose ist ein in Milch enthaltener Zucker.

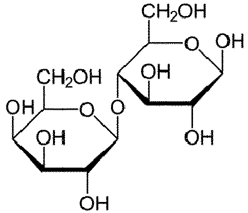

Das Disaccharid besteht aus den beiden Molekülen D-Galactose und D-Glucose, die über eine β-1,4-glycosidische Bindung miteinander verbunden sind. Nach IUPAC wird Lactose als 4-O-(β-D-Galactopyranosyl)-D-glucopyranose bezeichnet. Sie kommt als Hauptenergieträger in der Milch der Säugetiere vor. Lactose wird im Dünndarm vom Enzym Lactase verdaut, d. h. in Glucose und Galactose gespalten. In der Milch der Säugetiere sowie in Milcherzeugnissen macht Lactose fast den gesamten Anteil der Kohlenhydrate aus. Lactose, liefert Energie, unterstützt die Calcium-Resorption, hemmt Fäulnisbakterien im Darm des Menschen und begünstigt Bifidus-Bakterien (Bifidobacterium)

Molke ist die wässrige grünlich-gelbe Restflüssigkeit, die bei der Käseherstellung entsteht. Sie ist der flüssige Teil, der nach der Gerinnung der Milch zu Käse oder Quark abgesondert werden kann. Sauermolke entsteht, wenn Milch mit Milchsäurebakterien behandelt wird.

Milchpermeate insbesondere auf Basis von Magermilch sind seit langem bekannt. Es handelt sich um wässrige Flüssigkeiten, die bei der Filtration anfallen und bei der dem Einsatzprodukt Eiweiß und Milchfett entzogen wird. Zurück bleiben Vitamine, Mineralstoffe und Lactose. Für die Gewinnung von Milchpermeaten eignen sich grundsätzlich alle bekannten Filtrationsverfahren, als da sind:, Mikrofiltration, Ultrafiltration, Nanofiltration, oder eine Kombination dieser Schritte.

Es empfiehlt sich, Milchzuckerlösungen mit einer ausreichend hohen Feststoffmenge (synonym: Trockenmasse) einzusetzen, um das erfindungsgemäße Verfahren mit ökonomisch sinnvollen Umsätzen und Ausbeuten durchführen zu können. Hierzu eignen sich Lösungen, die eine Feststoffmenge von etwa 25 bis etwa 50 Gew.-% und vorzugsweise etwa 30 bis etwa 35 Gew.-% aufweisen. Gegebenenfalls können technische Milchzuckerlösungen beispielsweise durch Umkehrosmose ("reverse osmosis RO") entsprechend aufkonzentriert werden.

### Entkeimung

Im ersten Schritt des erfindungsgemäßen Verfahrens werden die wässrigen Milchzuckerlösungen entkeimt. Darunter ist jeder Prozess zu verstehen, mit dem sich die Keimlast des natürlichen Ausgangsproduktes auf einen Wert vermindern lässt, der unter dem liegt, den die jeweiligen nationalen Prüfstellen als Schwelle für die Zulassung als Lebensmittel festgelegt haben. In der Regel werden die Milchzuckerlösungen auf unter 1.000 Keime/mL entkeimt, vorzugsweise auf unter 500 Keime/mL und insbesondere etwa 10 bis etwa 50 Keime/mL. Die bevorzugte Entkeimungsmethode ist eine Hochtemperaturbehandlung, bei der die Lösungen einer Temperatur im Bereich von etwa 70 bis etwa 150 °C, vorzugsweise etwa 90 bis etwa 120 C über etwa 3 bis etwa 300 Sekunden, vorzugsweise etwa 50 bis etwa 200 Sekunden ausgesetzt werden. Eine Entkeimung kann entfallen, wenn bereits eine entkeimte Milchzuckerlösung eingesetzt wird.

### Enzymatische Transgalactosilierung

Die entkeimten Produkte werden im zweiten Verfahrensschritt einer enzymatischen Transgalactosilierung unterworfen. Hierunter versteht man die Übertragung von Galactoseeinheiten unter Aufbau eines oligomeren Zuckers in Gegenwart geeigneter Enzyme, in diesem Fall von beta-Galactosidasen, wobei Enzyme aus *Aspergillus oryzae, Bacillus circulans* oder Mischungen von beiden gemeinsam bzw. nacheinander zum Einsatz kommen.

Aspergillus oryzae, richtiger *Aspergillus flavus* var. *Oryzae* ist ein Schimmelpilz (Gießkannenschimmel), der in der japanischen Küche eine große Rolle spielt. Er ist der wichtigste unter den Ko̅ji-Pilzen Er wird vor allem benutzt, um Soja in Feststoff-Bioreaktoren zu fermentieren und so Miso und Sojasauce zu erzeugen.

*Bacillus circulans* ist eine Bakterienspezies, die sich zirkulär auf Nährmedien ausbreitet, woher auch ihr Namen stammt. Es handelt sich um anaerob wachsende, Gram variable stäbchenförmige, bewegliche Zellen, die 0,5 bis 1 µm breit und 3,5 µm lang sind. Das Bakterium fermentiert Pentosen, Hexosen, Hexitole und Disaccharide. *Bacillus circulans* kommt im Darm von pflanzenfressenden Fischen vor und unterstützt dort durch die Ausscheidung von Cellulasen die Verdauung.

Wie alle Enzyme weisen auch die beta-Galactosidasen ein vergleichsweise enges Temperatur- und pH-Wertintervall auf, in dem sie ihre optimale Leistung entfalten; diese sind dem Fachmann notorisch bekannt.

Mit *Aspergillus oryzae* wird die Reaktion daher vorzugsweise bei einer Temperatur im Bereich von etwa 50 bis etwa 60 °C und einem pH-Wert von etwa 4 bis etwa 5, bei Zugabe von Enzymen aus *Bacillus circulans* bei einer Temperatur im Bereich von etwa 45 bis etwa 55 °C und einem pH-Wert von etwa 5,5 bis etwa 6,5 durchführt. Bei Einsatz von Aspergillus oryzae wird daher auch von einem "sauren Verfahren", bei Bacillus circulans von einem "neutralen Verfahren" gesprochen.

Eine Besonderheit bei der grundsätzlichen Bildung von Galactooligosacchariden besteht darin, dass sich der Kettenaufbau nicht stetig fortsetzt, sondern nach einer Zeit an Geschwindigkeit verliert, bis sogar die Konkurrenzreaktion, nämlich die Rückspaltung der GOS überwiegt. Es hat sich daher als vorteilhaft erwiesen, der enzymabhängigen Reaktionskinetik Rechnung zu tragen und die Transgalactosilierung über einen Zeitraum von etwa 30 bis etwa 120 min und insbesondere von etwa 60 bis etwa 90 min durchzuführen. Dabei gilt, dass Hohe initiale Lactosekonzentrationen die Hydrolyseneigung deutlich vermindern und niedrige Enzymkonzentration die Hydrolyse ebenfalls verzögern.

### pH-Shift

Die Transgalactosilierung wird vorzugsweise solange durchgeführt, bis die höchste GOS-Konzentration erreicht ist. Dieser durch Enzym und Reaktionsbedingungen bedingte Wert kann durch Probennahme verfolgt und damit für den Fachmann leicht ermittelt werden. Ist das Maximum der GOS-Bildung erreicht, muss die Aktivität der Enzyme sehr schnell gestoppt werden, um eine Rückspaltung zu vermeiden. Dies kann beispielsweise durch schnelle Hocherhitzung erfolgen, bei der allerdings das Enzymmaterial vollständig abgetötet wird. Die vorliegende Erfindung bevorzugt einen anderen Weg und führt die Enzyme aus ihren optimalen Reaktionsbedingungen, konkret wird der pH-Wert durch Zugabe von Basen gegenüber dem Optimum um mindestens zwei Einheiten heraufgesetzt oder durch Zugabe von Säuren um mindestens zwei Einheiten herabgesetzt. Dieser pH-Shift bringt die Reaktion zwar nicht schlagartig zum Erliegen, vermindert die Aktivität der Enzyme jedoch um 80 bis 90 %, was für die Anforderungen ausreicht, eine nennenswerte Rückspaltung zu verhindern. Hierzu reicht es, den pH auf Werte von mindestens 7, vorzugsweise 8 bis 12 und insbesondere 9 bis 10 zu erhöhen oder auf Werte von 2 bis 5, vorzugsweise 3 bis 4 herabzusetzen. Bei der Änderung des pH-Wertes ist zu berücksichtigen, dass die Enzyme nicht irreversibel inaktiviert werden und der entsprechende pH-Wert - speziell im sauren Bereich - eine spätere Nutzung des Produktes nicht behindert. Der pH-Shift kann durch Zugabe einer erforderlichen Menge üblicher anorganischer Basen, wie beispielsweise einer wässrigen NaOH, durch Mineralsäuren wie HCl oder organische Säuren, wie beispielsweise Milchsäure erfolgen. Eine Anhebung des pH-Wertes ist gegenüber einer Absenkung bevorzugt.

### Nachbehandlung

Im Anschluss an die Transgalactosilierung erfolgt die Nachbehandlung der Reaktionsprodukte durch versetzen der Mischung mit Lactosehydrolase und/oder einer Hefe, die Lactose zu CO₂ und Ethanol verstoffwechseln kann. Vorzugsweise wird als Hefe *Kluyvermomyces lactis* eingesetzt. Auch die Nachbehandlung wird innerhalb deren optimalen Temperatur- und pH-Wert-Intervalls der Hydrolase und/oder Hefe - in der Regel zwischen 28 und 37 C - über einen Zeitraum von mindestens 30 Minuten durchgeführt. Eine Inhibierung der Hydrolasen und/oder Hefen ist in diesem Fall nicht zwingend erforderlich, kann aber je nach weiterem Einsatzzweck Sinn machen.

### Abtrennung und Wiederverwendung der Enzyme

Die Abtrennung der Enzym- bzw. Enzym/Hefemasse erfolgt vorzugsweise durch Filtration und insbesondere durch Ultrafiltration, die ebenfalls vorzugsweise kontinuierlich durchgeführt wird. Wurden die für Transgalactosilierung verwendeten Enzyme lediglich inhibiert, empfiehlt es sich, die Filtration vor der Nachbehandlung durchzuführen, um die abgetrennte Enzymmenge wird wieder in den Reaktionskreislauf zurückzuführen. Dabei gelangen die Enzyme direkt wieder in den Temperatur-und pH-Wertbereich, in dem ihr Optimum sich befindet. Gegebenenfalls kann eine solche Menge frisches Enzym nachdosiert werden, so dass die Enzymaktivität über das kontinuierliche Verfahren hinweg konstant oder wenigstens annähernd konstant bleibt.

Wurden die Enzyme durch Temperaturbehandlung zerstört, können diese im Anschluss an die Nachbehandlung zusammen mit der Lactosehydrolase und den Hefen abgetrennt werden, vorzugsweise ebenfalls mit Hilfe einer Ultrafiltration. In beiden Fällen enthält man ein Permeat Pα sowie ein Retentat Ra, wobei die gewünschten GOS im Permeat Pα enthalten sind.

An die Ultrafiltration kann sich dann eine Nanofiltration anschließen, um den Anteil an Monosacchariden und niedrigen Oligosacchariden zu entfernen, wobei man ein Permeat P3 und ein Retentat R3 erhält. Die gewünschten GOS finden sich diesmal im Retentat R3.

### Konfektionierung: Konzentrierung und Trocknung

Um konfektionierfähige Produkte zu erhalten, wird entweder das Permeat P3 oder das Retentat R4 aus den beiden vorangegangenen Schritten getrocknet und zuvor gegebenenfalls aufkonzentriert.

Um die GOS-Konzentration zu erhöhen können die Permeate noch aufgereinigt werden, beispielsweise durch Elektrodialyse oder Membranverfahren, wie z.B. eine Umkehrosmose. Falls erforderlich, kann die Trockenmasse durch Eindampfen erhöht werden.

Die Trocknung erfolgt beispielsweise durch Lyophilisierung, vorzugsweise aber durch Sprühtrocknung, wobei die Temperatur im Einlass typischerweise etwa 180 bis etwa 260 °C und am Ausgang etwa 80 bis etwa 105 °C beträgt. Der Restwassergehalt beträgt dabei maximal 5 Gew.-% und vorzugsweise etwa 1 bis etwa 2 Gew.-%.

### GEWERBLICHE ANWENDBARKEIT

Ein weiterer Gegenstand der Erfindung betrifft die Verwendung der neuen Galactooligosaccharide sowie der Stoffe, die nach dem erfindungsgemäßen Verfahren erhalten werden, als Nahrungsmittelergänzungsstoffe sowie Produkte für die Tierernährung.

### BEISPIELE

### Beispiel 1

### Herstellung von GOS nach dem neutralen Verfahren ausgehend von Lactoselösung

1.000 kg einer 30 Gew.-%igen Lactoselösung wurde in einem Röhrenwärmeaustauscher über 120 Sekunden auf 98 C erhitzt und dabei entkeimt. Die entkeimte Lösung wurde auf 55 C abgekühlt, in einen Fermenter überführt, mit Hilfe von Milchsäure auf einen pH-Wert von 6,5 eingestellt, mit beta-Galactosidase *aus Bacillus circulans* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht. Der pH-Wert wurde durch Zugabe von 30 Gew.-%iger Natronlauge innerhalb von wenigen Minuten auf 10 erhöht, wodurch die Aktivität des Enzyms schlagartig um 80 % verringert wurde. Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 in den Fermenter zurückgeführt; um Verlust auszugleichen wurden bezogen auf die Ausgangsmenge 5 Gew.-% frisches Enzym zugefügt. Das Permeat P1 wurde mit Lactosehydrolase im Gewichtsverhältnis Enzym/Hefe:Substrat von 1:25 und bei 35 C etwa 10 Stunden gerührt. Der Reaktionsansatz wurde zu einer zweiten Ultrafiltrationseinheit geleitet, die mit ebenfalls mit einer Spiralwickelmembran mit einer Porengröße von 10 kDa versehen war. Die Enzym/Hefemasse wurde mit dem Retentat R1 abgetrennt und das Permeat P1 zu einer Nanofiltrationseinheit geleitet, die mit einer Keramikmembran mit einer Porenweite von 500 bis 1.000 Da versehen war. Mit dem Permeat P2 wurden die noch im Produkt enthaltenen Monosaccharide abgetrennt, während das Retentat R2 einer Umkehrosmoseeinheit zugeleitet wurde, die mit einem Konzentrierungsfaktor von 1:2 arbeitete. Das dabei anfallende Permeat P3 (also das Konzentrierungswasser) wurde in das Verfahren zurückgeleitet, das Retentat R3 (also das GOS-Konzentrat) wurde im Plattenwärmeaustauscher 30 Sekunden auf etwa 85 C erhitzt und über einen Turm versprüht. Es wurde ein weißes Pulver mit einem GOS-Gehalt von mehr als 75 Gew.-% erhalten, welches noch eine Restfeuchte von 1 Gew.-% aufwies. Der DP der GOS betrug etwa 3.

### Beispiel 2

### Herstellung von GOS nach dem sauren Verfahren ausgehend von Sauermolke

1.000 kg einer ca. 30 Gew.-%igen Sauermolke (pH = 5,1) wurde in einem Röhrenwärmeaustauscher über 120 Sekunden auf 98 C erhitzt und dabei entkeimt. Die entkeimte Lösung wurde auf 50 C abgekühlt, in einen Fermenter überführt, mit beta-Galactosidase aus *Aspergillus oryzae* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht. Anschließend wurde der Reaktionssatz für etwa 10 Minuten auf 90 C erhitzt und die Enzyme abgetötet. Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 abgetrennt. Das Permeat P1 wurde mit 0,1 Gew.-% der Hefe *Kluyveromyces lactis* versetzt und weitere 5 h bei 30 bis 35 C gerührt. Anschließend wurde die Suspension einer zweiten Ultrafiltrationseinheit zugeführt, die Hefemasse als Retentat R2 abgetrennt und zurückgeführt und das Permeat P2 einer Umkehrosmoseeinheit zugeleitet, die mit einem Konzentrierungsfaktor von 1:4 arbeitete. Das dabei anfallende Permeat P3 (also das Konzentrierungswasser) wurde in das Verfahren zurückgeleitet, das Retentat R3 (also das GOS-Konzentrat) wurde im Plattenwärmeaustauscher auf etwa 70 bis 85 °C erhitzt und über einen Turm bei 200 °C versprüht. Es wurde ein weißes Pulver mit einem GOS-Gehalt von mehr als 90 Gew.-% erhalten, welches noch eine Restfeuchte von 1 Gew.-% aufwies. Der DP der GOS betrug etwa 4.

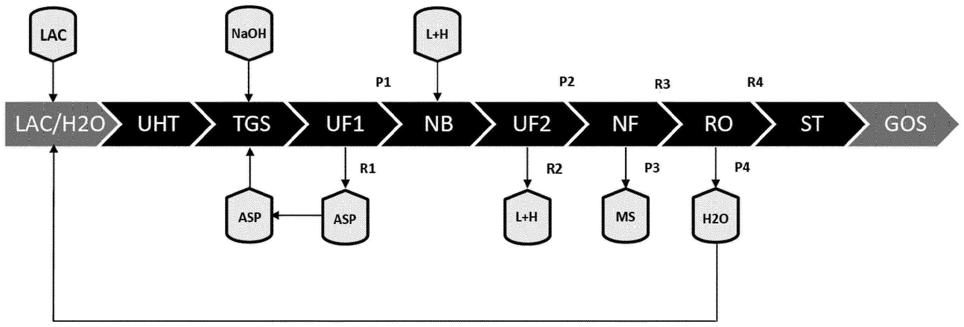

Beispiel 1 wird ferner an Hand eines Fließschemas gemäß **Abbildung 1** näher erläutert; dabei bedeuten die Abkürzungen:
- LAC: : Lactose
- LAC/H2O: : wässrige Lactoselösung
- UHT: : Ultrahocherhitzung
- TGS: : Transgalactosilierung
- ASP: : Aspergillus oryzae
- UF: : Ultrafiltration
- NB: : Nachbehandlung
- L+H: : Lactosehydrolase und Hefe
- NF: : Nanofiltration
- MS: : Monosaccharide
- RO: : Umkehrosmose
- ST: : Sprühtrockung
- GOS: : Galactooligosaccharide

## Patentansprüche

1. Galactooligosaccharide mit verminderten Anteilen an Monosacchariden und Lactose, dadurch erhältlich oder erhalten, dass man
(i) eine wässrige Milchzuckerlösung bereitstellt,
(ii) die Milchzuckerlösung entkeimt,
(iii) den in der entkeimten Milchzuckerlösung aus Schritt (ii) vorhandenen Milchzucker unter Zugabe mindestens einer beta-Galactosidase innerhalb deren optimalen Temperatur- und pH-Wert-Intervalls über einen Zeitraum von mindestens 30 Minuten einer Transgalactosilierung unter Erhalt einer Reaktionsmischung unterwirft;
(iv) die in der Reaktionsmischung aus Schritt (iii) enthaltene Enzymmasse inhibiert;
(v) die Reaktionsmischung aus Schritt (iv) mit einer Lactosehydrolase und/oder einer Hefe, die Lactose zu CO₂ und Ethanol verstoffwechseln kann, nachbehandelt;
(vi) das nachbehandelte Produkt aus Schritt (v) einer Filtration unterwirft und
(vii) das so erhaltene Filtrat aus Schritt (vi) konfektioniert.

2. Verfahren zur Herstellung von Galactooligosacchariden, umfassend die folgenden Schritte:
(i) Bereitstellen einer wässrigen Milchzuckerlösung;
(ii) Entkeimung der Milchzuckerlösung;
(iii) Transgalactosilierung der in der entkeimten Milchzuckerlösung aus Schritt (ii) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung;
(iv) Inhibierung der Enzymmasse in der Reaktionsmischung aus Schritt (iii)
(v) Nachbehandlung der Reaktionsmischung aus Schritt (iv) mit einer Lactosehydrolase und/oder einer Hefe, die Lactose zu CO₂ und Ethanol verstoffwechseln kann;
(vi) Filtration des nachbehandelten Produktes aus Schritt (v) sowie
(vii) Konfektionierung des Filtrates aus Schritt (iv).

3. Verfahren nach Anspruch 1, **dadurch gekennzeichnet, dass** man
(a) die Enzymmasse durch Einstellen eines pH-Wertes außerhalb des Aktivitätsoptimums ganz oder teilweise inhibiert;
(b) die Reaktionsmischung zusammen mit der inhibierten Enzymmasse einer Filtration unter Erhalt eines Retentats (R1) und eines Permeats (P1) unterwirft;
(c) die inhibierte Enzymmenge als Retentat (R1) abtrennt und zum Schritt (iii) zurückführt; und
(d) das die Galactooligosaccharide enthaltende Permeat (P2) zum Schritt (v) weiterleitet.

4. Verfahren nach mindestens einem der Ansprüche 2 und/oder 3, **dadurch gekennzeichnet, dass** man als Milchzuckerlösung eine wässrige Lactoselösung, Sauermolke oder Milchpermeat einsetzt.

5. Verfahren nach einem der Ansprüche 2 bis 4, **dadurch gekennzeichnet, dass** man Milchzuckerlösungen einsetzt, die eine Feststoffmenge von etwa 25 bis etwa 50 Gew.-% aufweisen.

6. Verfahren nach mindestens einem der Ansprüche 2 bis 5, **dadurch gekennzeichnet, dass** man die Entkeimung durch eine Hochtemperaturbehandlung bewirkt.

7. Verfahren nach mindestens einem der Ansprüche 2 bis 6, **dadurch gekennzeichnet, dass** man als beta-Galactosidase Enzyme aus *Aspergillus oryzae* und/oder *Bacillus circulans* einsetzt.

8. Verfahren nach Anspruch 7, **dadurch gekennzeichnet, dass** man die Transgalactosilierung durch Zugabe von *Aspergillus oryzae* bei einer Temperatur im Bereich von etwa 50 bis etwa 60 °C und einem pH-Wert von etwa 4 bis etwa 5 durchführt.

9. Verfahren nach Anspruch 7, **dadurch gekennzeichnet, dass** man die Transgalactosilierung durch Zugabe von *Bacillus circulans* bei einer Temperatur im Bereich von etwa 45 bis etwa 55 °C und einem pH-Wert von etwa 5,5 bis etwa 6,5 durchführt.

10. Verfahren nach mindestens einem der Ansprüche 2 bis 9, **dadurch gekennzeichnet, dass** man die Nachbehandlung mit der Hefe *Kluyvermomyces lactis* durchführt.

11. Verfahren nach mindestens einem der Ansprüche 2 bis 10, **dadurch gekennzeichnet, dass** man die Nachbehandlung innerhalb deren optimalen Temperatur- und pH-Wert-Intervalls der Hydrolase und/oder Hefe über einen Zeitraum von mindestens 30 Minuten durchführt.

12. Verfahren nach mindestens einem der Ansprüche 2 bis 9, **dadurch gekennzeichnet, dass** man das nachbehandelte Produkt einer Ultrafiltration unterwirft, bei der man das Permeat Pα und das Retentat Rα erhält.

13. Verfahren nach mindestens einem der Ansprüche 2 bis 10, **dadurch gekennzeichnet, dass** man Permeat P1 einer Nanofiltration unterwirft, bei der man das Permeat Pß und das Retentat Rß erhält.

14. Verfahren nach mindestens einem der Ansprüche 2 bis 13, **dadurch gekennzeichnet, dass** man das Retentat Rß aufkonzentriert und entwässert.

15. Verwendung der Galactooligosaccharide nach Anspruch 1 oder erhalten nach dem Verfahren gemäß den Ansprüchen 2 bis 14 als Nahrungsmittelergänzungsstoffe und Produkte für die Tierernährung.
